# EUROPEAN PATENT APPLICATION

(11) **EP 4 726 376 A1**
(43) Date of publication of application: **15.04.2026**
(21) Application number: 25774697.4
(22) Date of filing: 20.03.2025
(51) Int. Cl.: G01N 23/04, G01N 23/223

(54) **METALLIC FOREIGN MATERIAL ANALYSIS SYSTEM AND METALLIC FOREIGN MATERIAL ANALYSIS METHOD**

(30) Priority: 22.03.2024 KR 20240039773
(71) Applicant: LG Energy Solution, Ltd., Seoul 07335 (KR)
(72) Inventor: SON, Young Ki, Daejeon 34122 (KR); MOON, Hee Chul, Daejeon 34122 (KR)
(74) Representative: Goddar, Heinz J.
(86) International application number: PCT/KR2025/095029
(87) International publication number: WO 2025/198387

(57) **Abstract**

Disclosed herein relates to a metallic dust analysis system according to exemplary embodiments of the present disclosure including a dust analysis part, wherein the dust analysis part is configured to generate X-ray transmission images of each specimen and reference specimen, process the X-ray transmission images of the specimens to generate mapping image data of the specimens in which metal dusts having a diameter within a predetermined range are selectively displayed, and generate component analysis data for each of the metal dusts identified in the mapping image data.

## Description

### [Technical field]

This application claims the benefit of Korean Patent Application No. 10-2024-0039773, filed on March 22, 2024, the disclosure of which is incorporated herein by reference.

The present disclosure relates to a metallic dust analysis system and method that can detect only metallic dust among various dust scattered in a manufacturing facility or manufacturing site of an electrode, analyze the components of the detected metallic dust, quantitatively determine the distribution level of metallic dust, and qualitatively analyze the distribution level by type.

### [Background]

In recent years, rechargeable secondary batteries have been widely used as energy sources for wireless mobile devices. Secondary batteries are also attracting attention as an energy source for electric vehicles and hybrid electric vehicles, which are being proposed as a way to solve air pollution from conventional gasoline and diesel vehicles that use fossil fuels. Therefore, the types of applications using secondary batteries are diversifying due to their advantages, and it is expected that secondary batteries will be applied to many more fields and products in the future.

According to the shape of the battery case, these secondary batteries are classified into cylindrical batteries and prismatic batteries, in which the electrode assembly is embedded in a cylindrical or prismatic metal can, and pouch-type batteries, in which the electrode assembly is embedded in a pouch-type case of an aluminum laminated sheet, and the electrode assembly embedded in the battery case is a power generating device capable of charging and discharging and having a positive electrode, a negative electrode, and a separator structure interposed between the positive electrode and the negative electrode.

The positive electrode and the negative electrode are manufactured by applying a positive electrode slurry including a positive electrode active material and a negative electrode slurry including a negative electrode active material to the positive electrode current collector and the negative electrode current collector, respectively, to form a positive electrode active material layer and a negative electrode active material layer, a coating process to dry the positive electrode slurry and the negative electrode slurry, a rolling process to roll the dried electrode, and a notching and cutting process to form electrode tabs and to punching them into individual electrodes.

In various manufacturing facilities for the series of processes to manufacture electrodes, various dusts, both metallic and non-metallic, may be present, and these dusts may be incorporated into the electrodes during the manufacturing process of electrodes, and the incorporation of dusts into the electrodes may cause the performance of the battery to deteriorate. In particular, metal dusts can grow into dendrites, which can cause an internal short circuit in the secondary battery, resulting in failure, damage, or even ignition of the secondary battery.

Therefore, it is necessary to quantitatively identify the distribution level of metal dusts, especially among the various types of dusts present in manufacturing facilities such as coating facilities, drying facilities, rolling facilities, notching facilities, and slitting and cutting facilities, and to manage the level of dusts in manufacturing facilities. However, the prior art for detecting or analyzing metal dusts in the field of secondary batteries is limited to detecting and analyzing dusts in the product (battery), and there is no standardized method for quickly detecting and quantifying dusts, especially metal dusts, in the manufacturing facilities of electrodes. In addition, conventional methods for detecting and analyzing dusts require capturing them with tape and then analyzing them with a scanning electron microscope (SEM) or x-ray fluorescence (XRF), which is very time-consuming, unclear in identifying the metal composition, and inaccurate in measuring the size of the dusts.

Therefore, there is a need to develop a standardized method for detecting metal dusts and an analysis method including the same for improving the quality of electrodes.

### [Prior Art]

(Patent document 0001) Korean Public Patent No. 10-2022-0111364

### [Summary]

### [Technical Problem]

The technical problem that the present disclosure seeks to solve is to provide a standardized analysis system and analysis method for selectively detecting metallic dust having a predetermined range of sizes, quantifying them, and analyzing the components of the detected dust among various types of dust present in electrode manufacturing facilities or electrode manufacturing sites.

The present disclosure also aims to provide an analysis system and an analysis method that automates the process of detecting and quantitatively/qualitatively analyzing metallic dust.

### [Technical Solution]

According to exemplary embodiments of the present disclosure, a metallic dust analysis system is provided. The analysis system includes a dust analysis part configured to generate X-ray transmission images of each specimen and reference specimen, generate mapping image data of the specimens, in which metal dusts having a diameter within a predetermined range are selectively displayed by processing the X-ray transmission images of the specimens, and generate component analysis data for each of the metal dusts identified in the mapping image data.

In exemplary embodiments, the dust analysis part may be configured to utilize X-ray transmission image information of a reference specimen in establishing a numerical range of metallic dust diameters to be detected in the specimen.

In exemplary embodiments, the dust analysis part may be configured to image process the X-ray transmission image of the specimen based on the size information of the X-ray transmission image of the reference specimen.

In exemplary embodiments, the dust analysis part may be configured to filter out metallic dust having a diameter outside a predetermined range from the X-ray transmission image of the specimen, based on the size information of the X-ray transmission image of the reference specimen.

In exemplary embodiments, the dust analysis part may be configured to generate a high magnification X-ray transmission image or a high magnification mapping image data.

In exemplary embodiments, the dust analysis part may be configured to assign an identification code to each of the metallic dust screened in the mapping image data, and to generate the component analysis data per identification code.

In exemplary embodiments, the dust analysis part may be configured to automatically calculate a number of metallic dust and/or a respective size of the metallic dust from the mapping image data.

In exemplary embodiments, the component analysis data may include an X-ray fluorescence spectrum.

In exemplary embodiments, the dust analysis part may include:
a stage configured to hold the specimen and the reference specimen;
an X-ray transmission image generation module configured to irradiate the specimen and the reference specimen fixed on the stage with X-rays, detect an X-ray transmission signal transmitted through them, and generate an X-ray transmission image based on the detected signal;
an image processing module configured to image process the X-ray transmission image to generate the mapping image data; and
a component analysis module configured to generate component analysis data based on X-ray fluorescence analysis for each of the metallic dust visible in the mapping image data.

The analysis system according to the exemplary embodiments may further include: a database part for storing various data received from the dust analysis part; an input part configured to input various conditions for detection, image processing, component analysis, and data processing of metallic dusts by the dust analysis part; and an output part configured to output the mapping image data and component analysis data.

In exemplary embodiments, the reference specimen is a metal, wherein the specimen is a trap paper onto which dust captured from a manufacturing facility or manufacturing site of an electrode has been transferred, wherein the inspection may be configured to be performed in a state in which the reference specimen and the specimen are disposed in the same plane so that they do not overlap.

According to other embodiments of the present disclosure, a metallic dust analysis method is provided. The metallic dust analysis method includes: producing a specimen by capturing dust from a manufacturing facility or manufacturing site of an electrode; generating respective X-ray transmission images of a reference specimen and specimen using a dust analysis system using X-rays; selecting metallic dusts having a predetermined range of diameters from the X-ray transmission images of the specimen using the X-ray transmission image information of the reference specimen, and generating a mapping image data of the specimen in which the selected metallic dust is identified; and performing a component analysis on each of the metallic dust identified in the mapping image data to collect component analysis data.

In exemplary embodiments, the process of producing the specimen may include: a primary capture process of rolling a dust cleaning roller over a surface of a test article and capturing dust present on the surface of the test article onto the dust cleaning roller; a secondary capture process of transferring the dust adhering to the dust cleaning roller to a trap paper by secondarily rolling the primarily rolled dust cleaning roller onto the trap paper. Here, an adhesive component of the trap paper has a greater adhesive force than an adhesive component of the dust cleaning roller.

In exemplary embodiments, the dust analysis system includes a dust analysis part, wherein the dust analysis part may be configured to generate X-ray transmission images of each specimen and reference specimen, generate mapping image data of the specimens, in which metal dusts having a diameter within a predetermined range are selectively displayed by processing the X-ray transmission images of the specimens, and generate component analysis data for each of the metal dusts identified in the mapping image data.

In exemplary embodiments, the dust analysis part may be configured to image process the X-ray transmission image of the specimen based on the size information of the X-ray transmission image of a reference specimen.

In exemplary embodiments, the component analysis data may include an X-ray fluorescence spectrum.

### [Advantageous Effects]

According to the present disclosure, there is provided a dust analysis system and method that automates the process of selecting metal dust having a predetermined range of sizes from among metal dust having various sizes, generating mapping image data indicative of the locations of the dust, and generating component analysis data for each of the metal dust from the mapping image data.

According to the present disclosure, there is provided a standardized method for quantifying the distribution level and determining the component of metal dust of various types and sizes present in an electrode manufacturing facility or manufacturing site.

According to the present disclosure, the component analysis is performed only for metal dusts with a predetermined range of sizes, and the analysis can be completed quickly, which is advantageous for timely response when analyzing the cause of defects.

According to the present disclosure, an identification code is assigned to each of the metal dust identified in the mapping image data, and component analysis data is generated and managed according to the identification code, thereby facilitating data management.

### [Brief Description of the Drawings]

FIG. 1 is a schematic diagram of an analysis system according to exemplary embodiments of the present disclosure.
FIG. 2 is a block diagram of a dust analysis part according to exemplary embodiments of the present disclosure.
FIG. 3 is an X-ray transmission image of a reference specimen generated according to exemplary embodiments of the present disclosure.
FIG. 4 is an enlarged view of FIG. 3.
FIG. 5 illustrates mapping image data according to exemplary embodiments.
FIG. 6 is a flowchart to illustrate an analysis method according to exemplary embodiments of the present disclosure.
FIG. 7 is a flowchart to illustrate the process of preparing a specimen according to exemplary embodiments of the present disclosure.
FIG. 8 is a diagram illustrating a primary capture process according to exemplary embodiments of the present disclosure.
FIG. 9 is a diagram illustrating a secondary capture process according to exemplary embodiments of the present disclosure.
FIG. 10 is a diagram illustrating how specimens and reference specimens are mounted on a stage according to exemplary embodiments of the present disclosure.

### [Description of Reference Numerals].

10: SPECIMEN, TRAP PAPER
20: REFERENCE SPECIMEN
100: METALLIC DUST ANALYSIS SYSTEM
110: DUST ANALYSIS PART
120: DATABASE PART
130: INPUT PART
140: OUTPUT PART

### [Best Mode for Carrying out the Invention]

Hereinafter, preferred embodiments of the present disclosure will be described in detail with reference to the accompanying drawings. It should be noted that the terms and words used in this specification and claims are not to be construed in their ordinary or dictionary sense, but rather in a sense and concept consistent with the technical idea of the disclosure, based on the principle that the inventor may define the concept of a term as he sees fit to best describe his disclosure.

Accordingly, it is to be understood that the embodiments described herein and the configurations shown in the drawings are only the most preferred embodiments of the disclosure and are not exhaustive of the technical ideas of the disclosure, and that there may be various equivalents and modifications that may be substituted for them at the time of filing.

Furthermore, in describing the present disclosure, specific descriptions of related known configurations or features are omitted where it is deemed that such detailed description would obscure the essence of the disclosure.

Because the embodiments of the present disclosure are provided to more fully explain the disclosure to those of ordinary skill in the art, the shapes and sizes of components in the drawings may be exaggerated, omitted, or shown schematically for clarity. Accordingly, the size or proportion of each component is not necessarily indicative of its actual size or proportion.

### Metallic dust analysis system

Hereinafter, a metallic dust analysis system (hereinafter referred to as the "analysis system") according to the present disclosure will be described in detail.

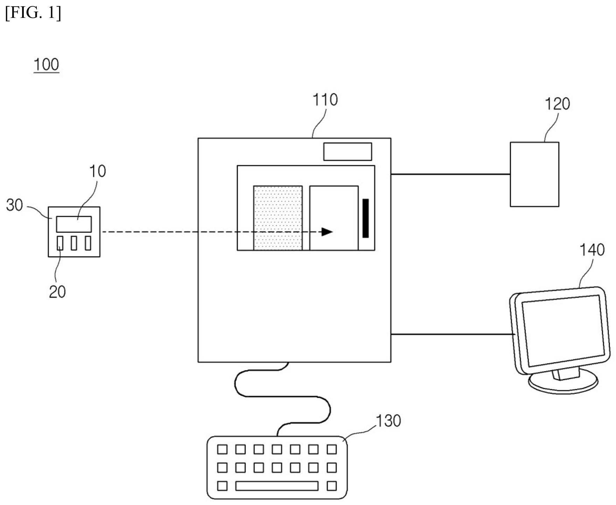

FIG. 1 is a schematic diagram of an analysis system according to exemplary embodiments of the present disclosure. Referring to FIG. 1, an analysis system 100 according to exemplary embodiments of the present disclosure may include a dust analysis part 110. The analysis system 100 may further include a database part 120, an input part 130, and an output part 140.

The dust analysis part 110 may be configured to generate respective X-ray transmission images of the specimen 10 and the reference specimen 20. The dust analysis part 110 may be configured to generate mapping image data of the specimens, in which metal dusts having a diameter within a predetermined range are selectively displayed by processing the X-ray transmission images of the specimens. The dust analysis part 110 may be configured to generate component analysis data for each of the metallic dust identified in the mapping image data. Here, the specimen 10 may include dusts captured from a manufacturing facility or manufacturing site of an electrode.

As shown above, the analysis system 100 according to the present disclosure may incorporate a series of tests to detect metallic dust present in the specimen, quantitatively determine the level of distribution of the detected metallic dust, and qualitatively analyze each component of the metallic dust. Furthermore, the analysis system 100 utilizes the transmission characteristics of X-rays, so it can accurately detect metal dust.

Furthermore, the analysis system 100 according to the present disclosure may be configured to utilize the X-ray transmission information of a reference specimen in establishing a numerical range of metallic dust diameters to be detected in the specimen. That is, the reference specimen 20 functions to provide a reference value for establishing a range of diameters of metallic dust to be detected in the specimen.

When the X-ray transmission image of the specimen 10 is unprocessed, the X-ray transmission image shows all of the different types and diameters of metal dusts, and it is not necessary to know the distribution level or composition of all sizes of metal dusts. Rather, it is of interest to know the distribution level or the respective components of metal dusts above a certain size, or of metal dusts having a predetermined range of sizes.

The analysis system 100 according to the present disclosure detects metal dusts in the specimen based on the transmission signal of X-rays, but selectively detects metal dusts having a certain size or a predetermined range of sizes, and performs component analysis only on these, so that the distribution level of significant metal dusts and their respective components can be determined very quickly, and a standardized method can be provided for determining the type and distribution level of dusts present in the electrode manufacturing facility or manufacturing site.

In exemplary embodiments, the specimen 10 may be a trap paper onto which dust captured from the electrode manufacturing facility or manufacturing site has been transferred. Details of how such trap paper is made will be described later.

In exemplary embodiments, the reference specimen 20 is not particularly limited as long as it is a metallic material. As a non-limiting example, the reference specimen 20 may be one metal selected from the group consisting of iron (Fe), copper (Cu), chromium (Cr), nickel (Ni), manganese (Mn), cobalt (Co), and aluminum (Al), or an alloy including two or more metals.

In exemplary embodiments, the reference specimen 20 may include a metal having a diameter corresponding to a range of diameters of the metal dust to be detected in the specimen 20. Specifically, if it is desired to detect metal dust having a diameter of 20 *µ*m or greater in the specimen 10, the reference specimen 20 may include metal having a diameter of 20 *µ*m. Alternatively, if it is desired to detect metal dust in the range of 15 to 20 *µ*m in the specimen, the reference specimen may include both 15 *µ*m diameter metal and 20 *µ*m diameter metal.

In exemplary embodiments, the analysis system 100 may be configured such that the test is performed with the reference specimen 20 and the specimen 10 disposed in the same plane such that they do not overlap each other.

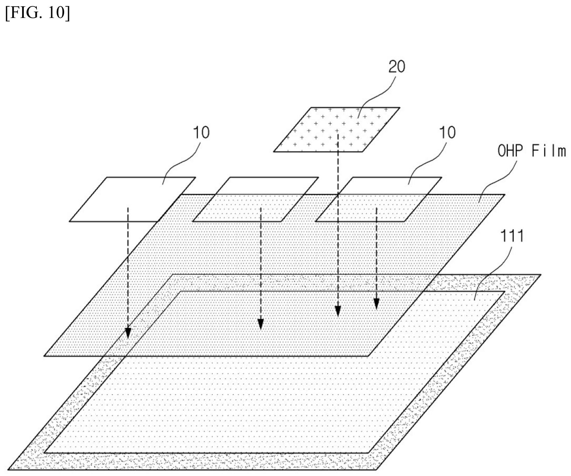

FIG. 10 is a diagram illustrating a method of mounting a specimen and a reference specimen on a stage according to exemplary embodiments of the present disclosure. Referring to FIG. 10, the specimen 10 and the reference specimen 20 are disposed on the stage 111, but are disposed in the same plane such that they do not overlap each other. This arrangement of the specimen 10 and the reference specimen 20 is intended to simultaneously acquire the respective X-ray transmission images of the reference specimen 20 and the specimen 10. Thus, the dust analysis part 110 can quickly filter out metallic dust in the X-ray transmission image of the specimen that do not have the desired size to be detected, based on the X-ray transmission information of the reference specimen.

In exemplary embodiments, the dust analysis part 110 may be configured to generate the X-ray transmission image by a digital method that converts the X-ray transmission signal into an electrical signal to generate an image. Thus, the analysis system 100 of the present disclosure can quickly filter out metallic dust that do not have the desired size to be detected, based on the number of pixels of the reference specimen in the digitized X-ray transmission image.

In exemplary embodiments, the dust analysis part 110 may be configured to image process the X-ray transmission image of the specimen based on the size information of the X-ray transmission image of the reference specimen. The size information of the X-ray transmission image of the reference specimen may be, as a non-limiting example, a number of pixels. Accordingly, mapping image data of specimens can be generated in which metal dusts having a predetermined diameter range are selectively indicated. In other words, the dust analysis part 110 may generate mapping image data of the specimen in which metal dusts having diameters in a predetermined range are selectively displayed by image processing to filter out metal dusts having diameters outside the predetermined range from the X-ray transmission image of the specimen 10, based on the size information of the X-ray transmission image of the reference specimen 20.

In exemplary embodiments, the dust analysis part 110 may be configured to generate a high magnification X-ray transmission image or a high magnification mapping image data. The metallic dust to be detected are typically several micrometers to tens of micrometers in size and are difficult to identify with the naked eye, but the high magnification images may allow the metallic dust to be identified.

In exemplary embodiments, the dust analysis part 110 may be configured to assign an identification code to each of the metallic dust identified in the mapping image data and to generate component analysis data for each identification code. Accordingly, the analysis system may collect and manage the component analysis data by identification code, facilitating data processing.

In exemplary embodiments, the dust analysis part 110 may be configured to automatically calculate the number of metallic dust and/or the respective size of the metallic dust from the mapping image data. Specifically, upon specifying a range in the mapping image data via the input part 130, the dust analysis part 110 may be configured to automatically calculate the number of metallic dust and/or the respective size of the metallic dust included in the specified range. The size of the metal dust may be, for example, an area of the metal dust, which may be calculated by a number of pixels. However, it is not limited thereto.

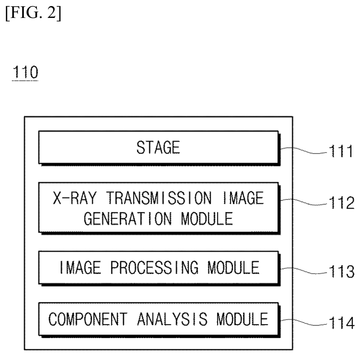

FIG. 2 is a block diagram of a dust analysis part 110 according to exemplary embodiments of the present disclosure. Referring to FIG. 2, the dust analysis part 110 according to exemplary embodiments may include a stage 111, an X-ray transmission image generation module 112, an image processing module 113, and a component analysis module 114.

In exemplary embodiments, the stage 111 may be configured to hold the specimen 10 and the reference specimen 20.

In exemplary embodiments, the X-ray transmission image generation module 112 may be configured to irradiate both the specimen 10 and the reference specimen 20 fixed on the stage 111 with X-rays, detect X-ray transmission signals transmitted through them, and generate respective X-ray transmission images of the specimen 10 and the reference specimen 20 based on the detected signals.

In exemplary embodiments, the X-ray transmission image generation module 112 may include an X-ray generator (not shown) that irradiates both the specimen 10 and the reference specimen 20 with X-rays; and a detector (not shown) configured to collect the X-rays that have penetrated the specimen 10 and the reference specimen 20 and generate an X-ray transmission image, which is an image that displays the collected energy corresponding to a location in a two-dimensional plane.

When the specimen 10 and the reference specimen 20 are irradiated with X-rays, the X-rays penetrate them, and the X-ray detector detects different energies depending on the type of material present in the specimen 10 and the reference specimen 20. These differences in the intensity of the X-ray transmission signals allow metallic dust to be detected in the specimen. The X-ray transmission image visualizing the detection results can then be used to determine the location, distribution, etc. of the metallic dust.

In some embodiments, the X-ray transmission image may be distinguished by the contrast between black and white. In a preferred embodiment, the detector may generate the X-ray transmission image digitally by converting the X-ray transmission signal into an electrical signal to generate an image. Specifically, the detector may generate the X-ray transmission image by digital radiography.

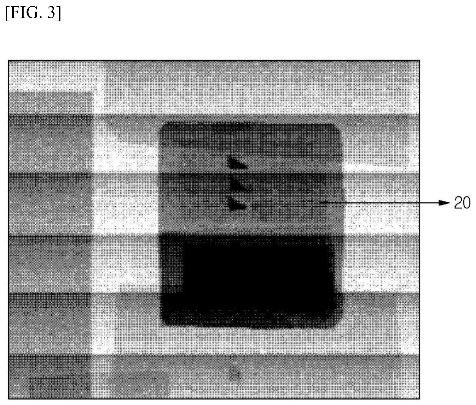

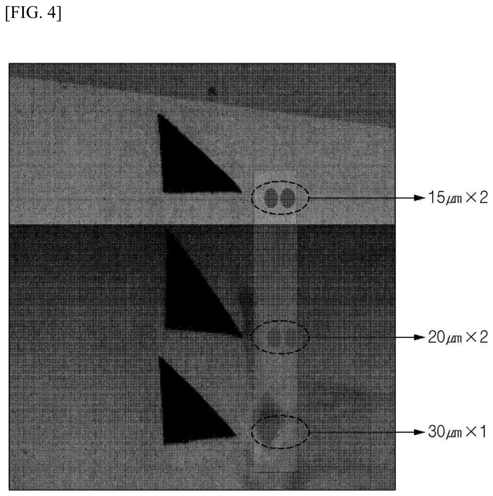

FIG. 3 is an X-ray transmission image of a reference specimen generated according to exemplary embodiments, and FIG. 4 is an enlarged view of FIG. 3. The reference specimen comprises two iron-chromium alloys having a diameter of 15 *µ*m, two iron-chromium alloys having a diameter of 20 *µ*m, and one iron-chromium alloy having a diameter of 30 *µ*m, the X-ray transmission images of which are shown in FIG. 4. The right triangular shape visible in the X-ray transmission images shown in FIGS. 3 and 4 is a kind of marker for visually marking a reference specimen.

In exemplary embodiments, the image processing module 113 may be configured to image process the X-ray transmission image to generate the mapping image data. That is, the mapping image data may be an image processing of the X-ray transmission image of the specimen under appropriate conditions. In exemplary embodiments, the image processing module 113 may be configured to image process the X-ray transmission image of the specimen based on the X-ray transmission image information of the reference specimen. Accordingly, the dust analysis part 110 according to the present disclosure may generate mapping image data in which, in the X-ray transmission image of the specimen, metal dust having a predetermined range of diameters, or metal dust having a size greater than or equal to the diameter of the reference specimen, are selectively displayed. As a result, the image processing module 113 may generate mapping image data in which metallic dust that are not detectable in the X-ray transmission image of the specimen are filtered out.

In addition, in exemplary embodiments, the image processing module 113 may be configured to assign an identification code to each of the metallic dust having a predetermined range of diameters. The identification code may be a letter, number, or symbol, or a combination of two or more of these. Accordingly, the analysis system of the present disclosure can facilitate processing and management of each component analysis data of the metallic dust identified by the identification code in the mapping image data.

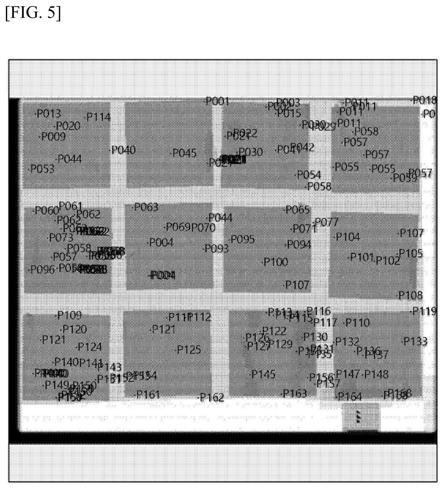

FIG. 5 illustrates mapping image data according to exemplary embodiments.

Referring to FIG. 5, each of the plurality of squares represents a specimen, i.e., FIG. 5 shows mapping image data for each of a plurality of specimens. Within each specimen, a plurality of points is marked, each of which is assigned an identification code. Thus, the operator can collect and manage the component analysis data by identification code.

In exemplary embodiments, the component analysis module 114 may be configured to generate component analysis data based on X-ray fluorescence analysis for each of the metallic dust identified in the mapping image data.

The X-ray fluorescence analysis is a technique that utilizes the unique interaction between fundamental X-rays and matter, and is an analytical technique that has been utilized in a variety of fields such as metal and alloy analysis, forensics, food analysis, environmental analysis, and the like. It is referred to as XRF analysis. X-ray fluorescence analysis can analyze substances on an elemental basis. When a specimen is analyzed by X-ray fluorescence, every element present emits a unique X-ray signal in the form of a spectrum. The specific X-rays of different elements can be separated into complete fluorescence energy spectra. Thus, the component analysis data may include the respective X-ray fluorescence spectra of the metallic dust.

The database part 120 may be configured to store various data received from the dust analysis part. The various data may include mapping image data of the specimen and component analysis data of each of the metallic dust. The component analysis data may be X-ray fluorescence energy spectra matched to respective identification code of the metallic dust.

In addition, in exemplary embodiments, the database part 120 may store the respective X-ray fluorescence energy spectra of various types of metal elements as reference data. Thus, by comparing the respective X-ray fluorescence spectra of the metallic dust detected in the specimen with the reference data, the constituent elements of the metallic dust can be determined.

The input part 130 may be configured to input various conditions for detection, image processing, component analysis, and data processing of the metallic dust by the dust analysis part. For example, the input part may be, but is not limited to, a keyboard, a mouse, or the like.

The output part 140 may be configured to output mapping image data and component analysis data. For example, the output part may be, but is not limited to, a monitor.

The analysis system according to the present disclosure may provide a simple analytical process and a standardized method for determining the distribution level of metallic dust in the electrode manufacturing facility or manufacturing site. Furthermore, since the analysis is not performed for all dusts, but only for those within a predetermined range of sizes, the analysis can be completed quickly, which is advantageous for timely response when analyzing the cause of defects.

### Metallic dust analysis method

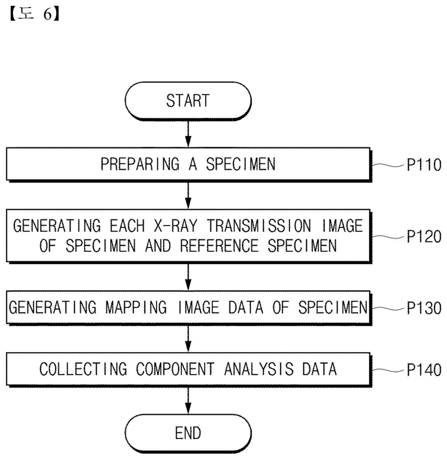

FIG. 6 is a flowchart to illustrate a metallic dust analysis method (hereinafter referred to as the "analysis method") according to exemplary embodiments of the present disclosure.

Referring to FIG. 6, an analysis method according to exemplary embodiments of the present disclosure may include preparing a specimen (P110), generating respective X-ray transmission images of the specimen and a reference specimen (P120), generating mapping image data of the specimen (P130), and collecting component analysis data (P140).

The process of preparing the specimen (P110) may be a process of preparing the specimen to capture dust from the test article to facilitate detection of metallic dust. The test article may refer to an electrode manufacturing facility or an electrode manufacturing site. An electrode manufacturing facility is any facility for manufacturing an electrode, including, but not limited to, a facility for manufacturing an electrode slurry, a facility for coating an electrode slurry, a facility for transferring a current collector sheet, a facility for drying an electrode, a facility for rolling an electrode, a facility for notching an electrode, and a facility for cutting an electrode.

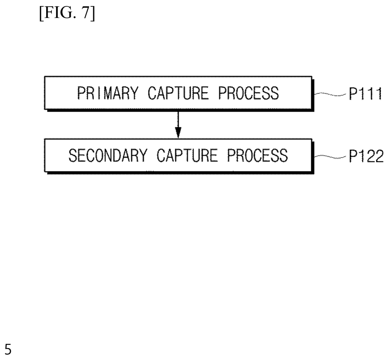

FIG. 7 is a flowchart to illustrate a process for preparing a specimen according to exemplary embodiments of the present disclosure. Referring to FIG. 7, the process of preparing a specimen P110 may include a primary capture process P111; and a secondary capture process P112.

The primary capture process P111 may be a process of primarily rolling the Dust Cleaning Roller over the surface of the specimen to capture dust present on the surface of the specimen with the Dust Cleaning roller.

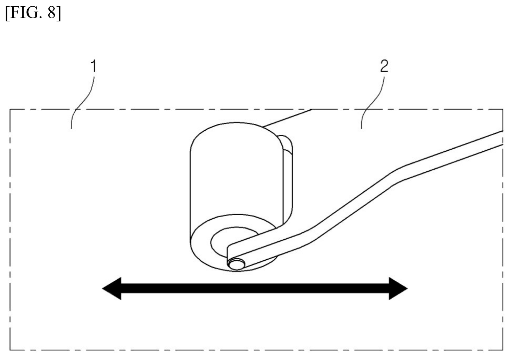

FIG. 8 is a diagram illustrating a primary capture process according to exemplary embodiments of the present disclosure. Referring to FIG. 8, in order to capture dust present on the test article, an operator rolls a dust cleaning roller 2 positioned on a surface of the test article 1. The dust cleaning roller 2 may be configured to adhere the dust present on the surface of the test article to the surface of the dust cleaning roller 2 by rolling. For example, the surface of the dust cleaning roller 2 may be coated with an adhesive material, so that the dust present on the rolling part of the test article can be attached to the surface of the dust cleaning roller 2.

The secondary capture process P112 may be a process of transferring the dust attached to the dust cleaning roller to the trap paper by secondarily rolling the primarily rolled dust cleaning roller onto the trap paper.

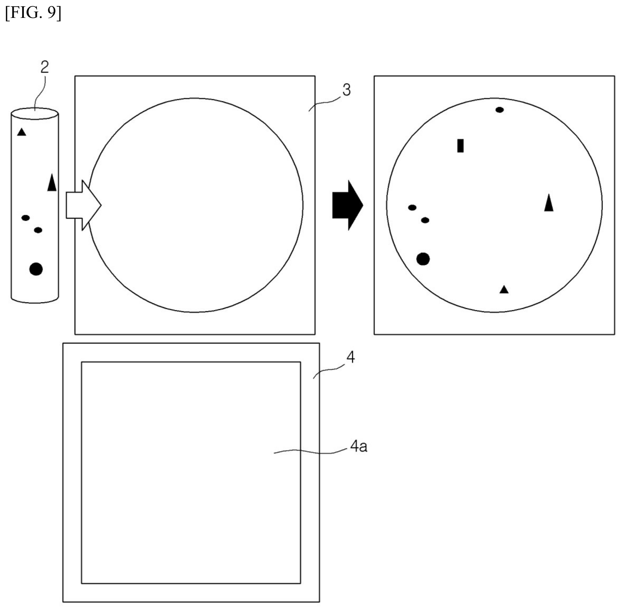

FIG. 9 is a diagram illustrating a secondary capture process according to exemplary embodiments of the present disclosure. Referring to FIG. 9, when the primarily rolled dust cleaning roller 2 is rolled onto the trap paper 3, the dust attached to the dust cleaning roller 2 is transferred to the trap paper 3 by the adhesive component of the trap paper 3. At this time, the dust is transferred to the trap paper 3 in a mono-layer.

In order for the dust attached to the surface of the dust cleaning roller 2 to be transferred to the trap paper 3, the surface of the trap paper 3 is coated with an adhesive component. The adhesive component is more adhesive than the adhesive component of the dust cleaning roller. Thus, when the dust cleaning roller 2 is rolled on the surface of the trap paper 3, the dust attached to the dust cleaning roller 2 can be transferred to the trap paper 3. The trap paper 3 onto which the dust is transferred can be kept closed with a trap cap 4 to prevent contamination of the trap paper. The trap cap 4 may comprise a transparent window 4a comprising a transparent film.

The process of generating the X-ray transmission images (P120) may be a process of generating the respective X-ray transmission images of the reference specimen and the specimen using a dust analysis system using X-rays.

The dust analysis system may be a dust analysis system as previously described. For example, the dust analysis system 100 may include a dust analysis part 110. The dust analysis part 110 may be configured to generate respective X-ray transmission images of a specimen and a reference specimen including dust captured from a manufacturing facility or manufacturing site of the electrode, and may be configured to generate mapping image data of the specimens, in which metal dusts having a diameter within a predetermined range are selectively displayed by processing the X-ray transmission images of the specimens, and may be configured to generate component analysis data for each of the metallic dust identified in the mapping image data.

The dust analysis system 100 may further include the previously described database part 120, input part 130, and output part 130.

The dust analysis part 110 may be configured to irradiate the specimen 10 and the reference specimen 20 with X-rays, detect X-ray transmission signals transmitted through them, and generate respective X-ray transmission images of the specimen 10 and the reference specimen 20 based on the detected signals. The method of detecting metallic dust using X-ray transmission images has the effect of enabling accurate and rapid detection.

The process of generating the mapping image data (P130) may be a process of using the X-ray transmission image information of the reference specimen to select metal dust having a predetermined range of diameters in the X-ray transmission image of the specimen, and generating the mapping image data in which the selected metal dust are identified.

In exemplary embodiments, the dust analysis part 110 may be configured to image process the X-ray transmission image of the specimen based on the size information of the X-ray transmission image of the reference specimen. That is, the dust analysis part 110 may generate mapping image data in which metallic dust having diameters outside a predetermined range are selectively displayed in the X-ray transmission image of the specimen through image processing that filters out metallic dust having diameters outside the predetermined range.

The process of collecting the component analysis data (P140) may be a process of collecting the component analysis data by performing a component analysis on each of the metal dust identified in the mapping image data. The component data may include an X-ray fluorescence spectrum.

The metallic dust analysis system has been described in detail previously and will not be duplicated herein.

The metallic dust analysis method according to the present disclosure provides a standardized method for quantitatively determining the level of distribution of metallic dusts among dusts present in an electrode manufacturing facility or manufacturing site. In addition, each X-ray transmission image of the specimen and the reference specimen is generated, and the X-ray transmission image information of the reference specimen is used to generate mapping image data of the specimen in which the metallic dust having the size to be detected are selectively displayed, so that the distribution level of significant metallic dust can be detected quickly and accurately. Furthermore, component analysis data is generated for each of the metal dust identified in the mapping image data, an identification code is assigned to each of the metal dust identified in the mapping image data, and component analysis data is generated and managed by the identification code, thereby facilitating data management.

The present disclosure has been described in more detail above with reference to the drawings and embodiments. However, it should be understood that the configurations described in the drawings or embodiments are only one embodiment of the disclosure and do not represent all of the technical ideas of the disclosure, and that there may be various equivalents and modifications that may be substituted for them at the time of filing the present application.

## Claims

1. A metallic dust analysis system comprising: a dust analysis part configured to generate X-ray transmission images of each specimen and reference specimen, generate mapping image data of the specimens, in which metal dusts having a diameter within a predetermined range are selectively displayed by processing the X-ray transmission images of the specimens, and generate component analysis data for each of the metal dusts identified in the mapping image data.

2. The metallic dust analysis system of claim 1, wherein
the dust analysis part is configured to utilize X-ray transmission image information of a reference specimen in establishing a numerical range of metallic dust diameters to be detected in the specimen.

3. The metallic dust analysis system of claim 1, wherein
the dust analysis part is configured to image process the X-ray transmission image of the specimen based on the size information of the X-ray transmission image of the reference specimen.

4. The metallic dust analysis system of claim 1, wherein
the dust analysis part is configured to filter out metallic dust having a diameter outside a predetermined range from the X-ray transmission image of the specimen, based on the size information of the X-ray transmission image of the reference specimen.

5. The metallic dust analysis system of claim 1, wherein
the dust analysis part is configured to generate a high magnification X-ray transmission image or a high magnification mapping image data.

6. The metallic dust analysis system of claim 1, wherein
the dust analysis part is configured to assign an identification code to each of the metallic dust screened in the mapping image data, and to generate the component analysis data per identification code.

7. The metallic dust analysis system of claim 1, wherein
the dust analysis part is configured to automatically calculate a number of metallic dust and/or a respective size of the metallic dust from the mapping image data.

8. The metallic dust analysis system of claim 1, wherein
the component analysis data comprises an X-ray fluorescence spectrum.

9. The metallic dust analysis system of claim 1, wherein
the dust analysis part comprises:
a stage configured to hold the specimen and the reference specimen;
an X-ray transmission image generation module configured to irradiate the specimen and the reference specimen fixed on the stage with X-rays, detect an X-ray transmission signal transmitted through them, and generate an X-ray transmission image based on the detected signal;
an image processing module configured to image process the X-ray transmission image to generate the mapping image data; and
a component analysis module configured to generate component analysis data based on X-ray fluorescence analysis for each of the metallic dust visible in the mapping image data.

10. The metallic dust analysis system of claim 1, further comprising:
a database part for storing various data received from the dust analysis part;
an input part configured to input various conditions for detection, image processing, component analysis, and data processing of metallic dusts by the dust analysis part; and
an output part configured to output the mapping image data and component analysis data.

11. The metallic dust analysis system of claim 1, wherein
the reference specimen is a metal, wherein
the specimen is a trap paper onto which dust captured from a manufacturing facility or manufacturing site of an electrode has been transferred, wherein
the inspection is configured to be performed in a state in which the reference specimen and the specimen are disposed in the same plane so that they do not overlap.

12. A metallic dust analysis method comprising:
producing a specimen by capturing dust from a manufacturing facility or manufacturing site of an electrode;
generating respective X-ray transmission images of a reference specimen and specimen using a dust analysis system using X-rays;
selecting metallic dusts having a predetermined range of diameters from the X-ray transmission images of the specimen using the X-ray transmission image information of the reference specimen, and generating a mapping image data of the specimen in which the selected metallic dust is identified; and
performing a component analysis on each of the metallic dust identified in the mapping image data to collect component analysis data.

13. The metallic dust analysis method of claim 12, wherein
the process of producing the specimen comprises:
a primary capture process of rolling a dust cleaning roller over a surface of a test article and capturing the dust present on the surface of the test article onto the dust cleaning roller;
a secondary capture process of transferring the dust adhering to the dust cleaning roller to a trap paper by secondarily rolling the primarily rolled dust cleaning roller onto the trap paper, wherein
an adhesive component of the trap paper has a greater adhesive force than an adhesive component of the dust cleaning roller.

14. The metallic dust analysis method of claim 12, wherein
the dust analysis system comprises:
a dust analysis part configured to generate X-ray transmission images of each specimen and reference specimen, generate mapping image data of the specimens, in which metal dusts having a diameter within a predetermined range are selectively displayed by processing the X-ray transmission images of the specimens, and generate component analysis data for each of the metal dusts identified in the mapping image data.

15. The metallic dust analysis method of claim 11, wherein
the dust analysis part is configured to image process the X-ray transmission image of the specimen based on the size information of the X-ray transmission image of a reference specimen.

16. The metallic dust analysis method of claim 11, wherein
the component analysis data comprises an X-ray fluorescence spectrum.
